# EUROPEAN PATENT SPECIFICATION

(11) **EP 2 393 779 B1**
(45) Date of publication and mention of the grant of the patent: **06.11.2013**
(21) Application number: 10738292.1
(22) Date of filing: 04.02.2010
(51) Int. Cl.: C07D 407/14, C07H 5/02, C07H 9/04, C07H 13/04, C07H 15/04, C07H 17/04

(54) **PROCESS FOR THE SYNTHESIS OF CLEISTANTHIN**
VERFAHREN ZUR SYNTHESE VON CLEISTANTHIN
PROCÉDÉ DE SYNTHÈSE DE CLEISTANTHINE

(30) Priority: 05.02.2009 IN MU02342009
(43) Date of publication of application: 14.12.2011
(73) Proprietor: Godavari Biorefineries Ltd., Fort Mumbai 400 001 Maharashtra (IN)
(72) Inventor: SINGH, Om, Vir, Thane West 400 610 Maharashtra (IN); TAPADIYA, Sarika, Madhusudan, Thane West 400 610 Maharashtra (IN); DESHMUKH, Rahul, Ganpat, Thane West 400 610 Maharashtra (IN)
(74) Representative: Kilger, Ute
(86) International application number: PCT/IN2010/000066
(87) International publication number: WO 2010/089778

(56) References cited:
- ANIANEYULU A S R ET AL: "New Lignans from the Heartwood of Cleistanthus collinus", TETRAHEDRON, ELSEVIER SCIENCE PUBLISHERS, AMSTERDAM, NL, vol. 37, no. 21, 1 January 1981 (1981-01-01), pages 3641-3652, XP002502258, ISSN: 0040-4020, DOI: 10.1016/S0040-4020(01)98893-3
- AYRES ET AL.: 'Lignans: Chemical, biological, and clinical propeties' CAMBRIDGE UNIVERSITY PRESS 1990, page 194, XP008156011
- LEWIS ET AL.: 'The phase transfer catalysed synthesis of isoflavone-O-glucosides' J CHEM SOC, PERKIN TRANS vol. 1, 1998, pages 2481 - 2484, XP008156007
- BRITO-ARIAS: 'Synthesis and Characterization of Glycosides' SPRINGER SCIENCE+BUSINESS MEDIA 2007, pages 1 - 351, XP008156000
- HUNTER: 'Towards the total synthesis of haplomyrtin' M.S. THESIS, WRIGHT STATE UNIVERSITY 07 June 2010, pages 1 - 61, XP008156001

## Description

### FIELD OF THE INVENTION

The present invention relates to a process for preparing a diphyllin glycoside, Cleistanthin, more particularly Cleistanthin A.

### DESCRIPTION OF THE BACKGROUND ART

Diphyllin belongs to the family of lignans. Diphyllin is a phenolic lignan lactone. The lignans are a group of chemical compounds found in plants, particularly in flax seed. Lignans are one of the major classes of phytoestrogens, which are estrogen-like chemicals and also act as antioxidants. Diphyllin exists in the glycosylated form. Cleistanthin is an example of a glycosylated diphyllin.

The structure of Cleistanthin A is as shown below:

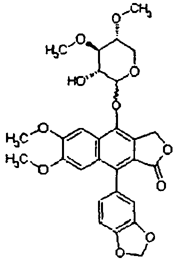

The glycosylated forms of lignan have been identified for a wide variety of activities. They have been associated with cytotoxic effects as well as antitumor activity and antiinflammatory effects.

The conventional process for the isolation of Cleistanthin A from *cleistanthus collinus* comprises the steps of treating the dried leaves of *cleistanthus collinus* with petroleum ether to obtain a defatted powder. The defatted powder is extracted with acetone to form a gummy mass, which on further treatment with benzene and chloroform gives a black residue. The spots are obtained on TLC plates, which on chromatogram with heptanes, chloroform and methanol gives Cleistanthin A in the form of blue fluorescent spots. Cleistanthin A is re-crystallized with acetone.

WO 2008058897 relates to a V-ATPase or gastric proton pump inhibiting lignan such as diphyllin and related glycosides and derivatives, for use as a medicament in the treatment of excessive osteoclast action or in the treatment of excessive gastric acidification, suitably of the general formula: wherein: R^{b} and R^{c} may together form an alkylene bridge; R^{d} and R^{e} also may together form an alkylene bridge; and each dotted bond independently is present or absent; and wherein the lactone ring is optionally opened and is optionally esterified. The compounds disclosed are of the general formula:

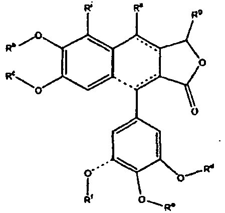

### SUMMARY OF THE INVENTION

It is an object of the present invention to prepare Cleistanthin A by a synthetic process.

An aspect of the present invention encompasses a process to prepare compound of formula I that is Cleistanthin A, wherein the process comprises the steps of reacting compound of formula II with compound of formula III in the presence of a first solvent, a quarternary ammonium salt and a first alkali to form compound of formula IV. The compound of formula IV is further treated with a second solvent and a second alkali to form compound of formula I.

It is an additional aspect of the present invention to provide a process for preparing compound of formula IV that is Cleistanthin A acetate. The process comprises the steps of reacting compound of formula II with compound of formula III in the presence of a solvent, a quarternary ammonium salt and an alkali to form compound of formula IV.

The structures of the compounds are represented below:

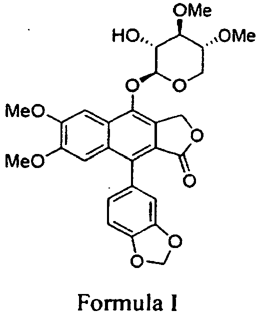

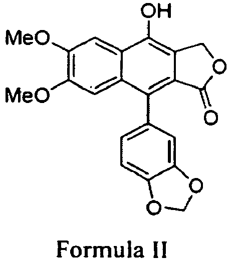

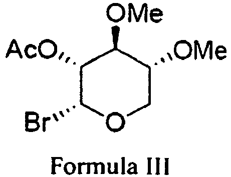

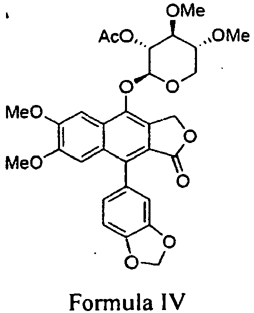

### DETAILED DESCRIPTION OF THE INVENTION

The present invention relates to process for preparing Cleistanthin A and its acetate salt that is Cleistanthin A acetate.

An embodiment of the present invention provides a process for preparing compound of formula I that is Cleistanthin A. The reaction scheme of the process is as follows:

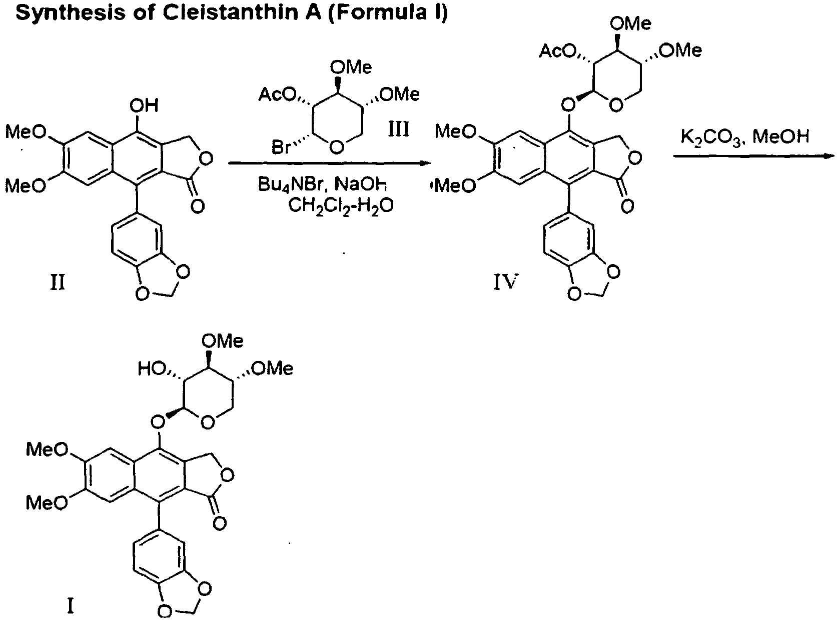

The process comprises the steps of reacting a compound of formula II with a compound of formula III in the presence of a first solvent, a quarternary ammonium salt and a first alkali to give a compound of formula IV that is Cleistanthin A acetate. The first solvent used to carry out the reaction is dichloromethane. The quarternary ammonium salt is tetrabutyl ammonium bromide. The first alkali used is sodium hydroxide. Compound of formula IV is further treated with a second alkali in the presence of a second solvent to form compound of formula I that is Cleistanthin A. The reaction mixture is stirred at room temperature for nearly 30 minutes. The second alkali used is potassium carbonate. The second solvent used is methanol.

The compound of formula I is (9-(1,3-Benzodioxol-5-yl)-4-((3,4-di-O-methyl-D-xylopyranosyl)oxy)-6,7-dimethoxynaphtho(2,3-c)furan-1(3H)-one,) that is Cleistanthin A. The compound of formula II is 9-(3', 4'-Methylenedioxyphenyl)-4-hydroxy-6,7-dimethoxynaphtho[2,3-*c*]furan-1(3*H*)-one. The compound of formula III is 2-*O*-Acetyl-3, 4-dimethoxy-α-D-bromoxylopyranose. The compound of formula IV is (9-(1,3-Benzodioxol-5-yl)-4-((3,4-di-O-methyl-D-xylopyranosyl)acetate)-6,7-dimethoxynaphtho(2,3-c)furan-1(3H)-one,) that is Cleistanthin A acetate.

The process for preparing Cleistanthin A is simple and economically viable with a high yield of 96% of Cleistanthin A.

Another embodiment of the present invention relates to a process for preparing compound of formula IV that is Cleistanthin A acetate. The reaction scheme of the process is as follows:

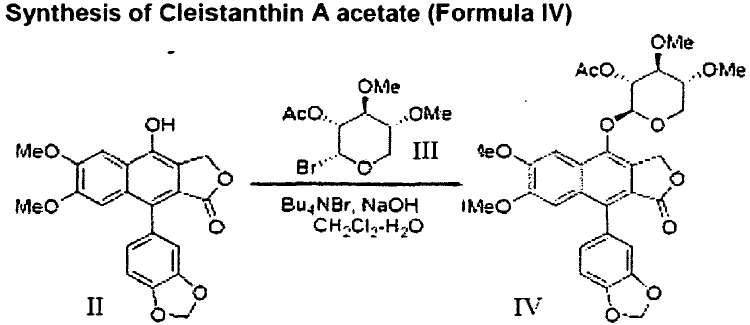

The process comprises the steps of reacting a compound of formula II with a compound of formula III in the presence of a solvent, a quarternary ammonium salt and an alkali to give a compound of formula IV that is Cleistanthin A acetate. The solvent used to carry out the reaction is dichloromethane. The quarternary ammonium salt is tetrabutyl ammonium bromide. The alkali used is sodium hydroxide.

The compound of formula II is 9-(3', 4'-Methylenedioxyphenyl)-4-hydroxy-6,7-dimethoxynaphtho[2,3-*c*]furan-1(3*H*)-one. The compound of formula III is 2-*O*-Acetyl-3, 4-dimethoxy-α-D-bromoxylopyranose. The compound of formula IV is that is Cleistanthin A acetate ((9-(1,3-Benzodioxol-5-yl)-4-((3,4-di-O-methyl-D-xylopyranosyl)acetate)-6,7-dimethoxynaphtho(2,3-c)furan-1(3H)-one,).

According to another embodiment of the present invention a process for preparing compound of formula II is represented as follows:

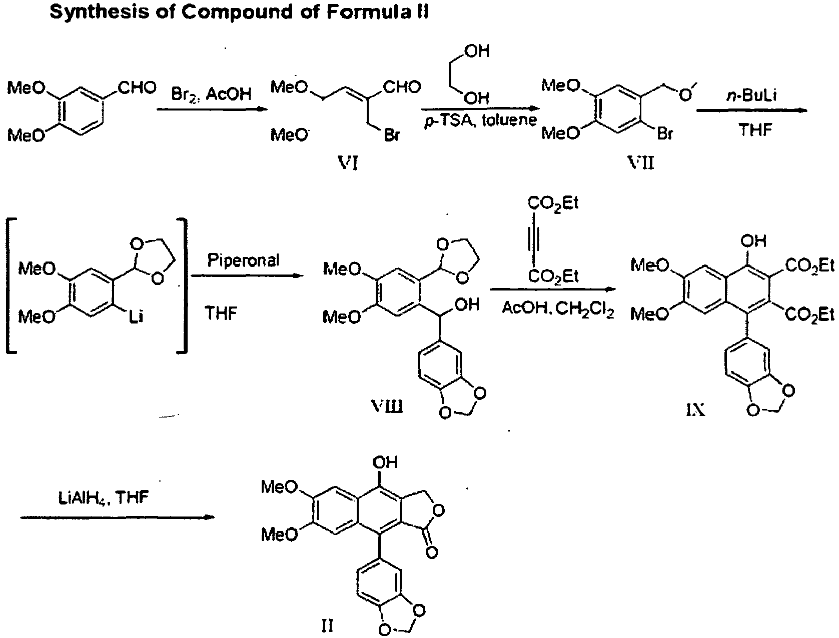

The process comprises the step of halogenating a compound of formula V to give a compound of formula VI. Halogenation is carried out in the presence of bromine and acetic acid. The reaction is carried out at room temperature for 3-5 hours. Compound of formula VI is refluxed with p-ethylene glycol in the presence of p-toluene sulphonic acid to give compound of formula VII. Compound of formula VII is treated with n-butyl lithium in the presence of tetrahydrofuran and piperonal to form compound of formula VIII.The temperature is maintained to -70 to -80°C- for 1-5 hours. Compound of formula VIII is further heated with diethyl acetylenedicarboxylate in the presence of acetic acid and methylene dichloride to give compound of formula IX. The temperature is maintained from 130-150°C for 1-2 hours. Compound of formula IX is further treated with lithium aluminium hydride in the presence of tetrahydrofuran to give compound of formula II. The temperature is maintained to 0°C. The reaction time is from 2-3 hours. The compound of formula V is veratraldehyde or 4,5-dimethoxybenzaldehyde. The compound of formula VI is 2-Bromo-4,5-dimethoxybenzaldehyde. The compound of formula VII is 2-(2-Bromo-4,5-dimethoxyphenyl)-1,3-dioxolane. The compound of formula VIII is (2-(1,3-Dioxolan-2-yl)-4,5-dimethoxyphenyl)(benzo[d][1,3]dioxol-5-yl)-methanol. The compound of formula IX is Diethyl 1-(3',4'-methylenedioxyphenyl)-4-hydroxy-6,7-dimethoxy-naphthalene-2,3-dicarboxylate.

According to another embodiment of the present invention a process for preparing compound of formula III is represented as follows:

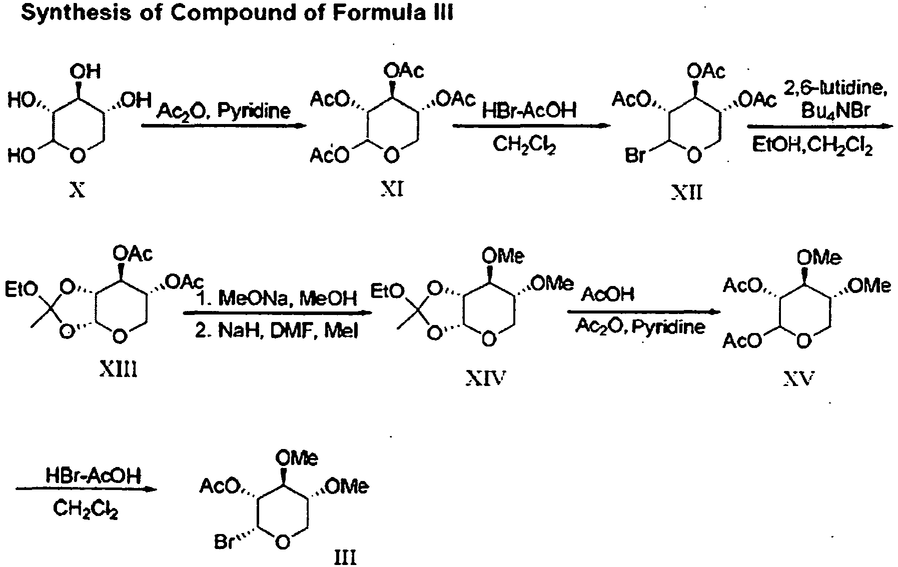

The process comprises the step of reacting a compound of formula X with pyridine. Acetic anhydride is added to the mixture to result in compound of formula XI. The temperature of the reaction mixture is maintained at 0°C for 4-6 hours. Compound of formula XI is further treated with dichloromethane. The reaction mixture is cooled to 0°C. To this cold solution hydrogen bromide in acetic acid is added to form a compound of formula XII. The reaction is carried out for 1-3 hours. Compound of formula XII is treated with 2,6 lutidine, tetrabutyl ammonium bromide, anhydrous dichloromethane and ethanol at room temperature to form a compound of formula XIII. Compound of formula XIII is treated with methanol and sodium methoxide to obtain a residue. The reaction is carried at room temperature for 1-2 hours. The residue is dissolved in dimethyl formamide and the mixture is cooled to 0 deg C. To the resulting solution sodium hydride is added to form a suspension. Methyl iodide is further added to the resulting suspension to form compound of formula XIV. Compound of formula XIV is dissolved in acetic acid and the reaction mixture is concentrated to obtain a residue. The temperature is maintained at 0°C for 1-2 hours. The residue obtained is further treated with acetic anhydride and pyridine at room temperature to form compound of formula XV. Compound of formula XV is dissolved in dichloromethane. The reaction mixture is cooled to 0°C. To the cooled solution, hydrogen bromide in acetic acid is added to from compound of formula III. The reaction time is 2-3 hours.

The compound of formula X is D-xylose. The compound of formula XI is Tetra-O-acetyl-D-xylopyranose. The compound of formula XII is 2,3,4-Tri-O-acetyl-α-D-bromoxylopyranose. The compound of formula XIII is 3,4-Di-O-acetyl-1,2-O-(1-ethoxyethylidene)-D-xylopyranose. The compound of formula XIV is 1,2-O-(1-Ethoxyethylidene)-3,4-dimethoxy-D-xylopyranose. The compound of formula XV is 1,2-Di-O-acetyl-3,4-dimethoxy-D-xylopyranose.

Cleistanthin A is used to treat diseases such as cancer, excessive gastric acidification, excessive osteoclast action, treatment and prophylaxis of protein kinase C (PKC) related condition in mammals.

The following example illustrate the invention, but is not limiting thereof.

### Example 1: Preparation of Cleistanthin A of formula I

Cleistanthin A was prepared by the following reaction steps:

### I. Synthesis of Cleistanthin A acetate (Formula IV)

To a 50 mL round bottom flask, 9-(3',4'-Methylenedioxyphenyl)-4-hydroxy-6,7-dimethoxynaphtho[2,3-*c*]furan-1(3*H*)-one (formula II; 0.30 g, 0.788 mmole), 2-*O*-Acetyl-3,4-dimethoxy-α-D-bromoxylopyranose (formula III, 0.446 g, 1.576 mmole) and tetrabutyl ammonium bromide (0.254 g, 0.788 mmole) were taken in dichloromethane (20 mL) with stirring. To this suspension was added 2M NaOH (3 mL) solution and stirring was continued for 2 h at room temperature. After the completion of reaction as judged by TLC (1:9, EtOAc:DCM), the reaction mixture was extracted with dichloromethane (4 x 20 mL). The combined organic layer washed with 10% NaOH solution (3 x 15 mL) followed by water (2 x 10 mL) and dried over anhydrous sodium sulfate. Inorganic salts were filtered off; filtrate was concentrated under reduced pressure and crude mass which was purified by column chromatography using EtOAc: dichloromethane (04:96) as eluent to Cleistanthin A acetate (formula IV) as white solid.

The yield and NMR details of compound of Cleistanthin A acetate were as follows: Yield: 0.240 g (52%)
¹HNMR (CDCl₃, 300 MHz): δ = 7.59 (s, 1H), 7.04 (s, 1H), 6.94 (d, 1H, *J* = 7.8 Hz), 6.81-6.76 (m, 2H), 6.06 (d, 1H, *J* = 13.8 Hz), 6.05 (d, 1H, *J* = 13.8 Hz), 5.47 (d, 1H, *J* = 16.2 Hz), 5.39 (d, 1H, *J* = 14.8 Hz), 5.33 (t, 1H, *J* = 7.2 Hz), 5.10 (d, 1H, *J* = 6.9 Hz), 4.15 (dd, 1H, J = 6.0, 13.2 Hz), 4.07 (s, 3H), 3.79 (s, 3H), 3.60 (s, 3H), 3.50 (s, 3H), 3.46-3.32 (m, 3H), 2.15 (s, 3H). ¹³CNMR (300 MHz, CDCl₃): δ = 169.68, 169.47, 151.83, 150.35, 147.51, 144.05, 135.57, 130.63, 128.37, 126.12, 125.94, 123.57, 119.25, 110.70, 108.20, 106.10, 101.21, 100.80, 100.58, 81.21^{**}, 81.16^{**}, 77.95, 71.44^{*}, 71.40^{*}, 66.94, 62.77, 59.90, 58.53, 56.22, 55.81, 21.14.
^{*} Signals of rotamers of same carbons due restricted rotation created by glycosidation.
^{**}Signals of rotamers of same carbons due restricted rotation created by glycosidation

### II. Synthesis of Cleistanthin A (Formula I)

To a solution of Cleistanthin A acetate (formula IV, 0.20 g , 0.343 mmole) in methanol (7.5 mL) was added solid anhydrous K₂CO₃ (0.0925 g .675 mmol) and reaction mixture was stirred at room temperature for 30 min. After completion of reaction as judged by TLC (5:5, EtOAc:Hexane), methanol was removed under reduced pressure, water was added and extracted with CH₂Cl₂ (2 x 25 mL). Organic layer was dried over anhydrous sodium sulfate, filtered and concentrated to get Cleistanthin A (formula I) as white fluffy solid.

The yield and NMR details of compound of Cleistanthin A were as follows:
Yield: 179 mg (96%)
¹HNMR (CDCl₃, 300 MHz): δ = 7.92 (s, 1H), 7.05 (d, 1H, *J* = 1.5 Hz), 6.94 (dd, 1H, J = 1.2, 7.8 Hz), 6.83-6.78 (m, 2H), 6.07 (d, 1H, J= 14.1 Hz), 6.06 (d, 1H, J= 14.4 Hz), 5.49 (d, 1H, *J* = 14.7 Hz), 5.42 (d, 1H, *J =* 14.7 Hz), 5.10 (d, 1H, *J* = 5.7 Hz), 4.10 (dd, 1H, *J* = 2.4, 12.0 Hz), 4.04 (s, 3H), 3.95-3.88 (m, 1H), 3.80 (s, 3H), 3.68 (s, 3H), 3.49 (s, 3H), 3.45 (dd, 1H, *J =* Hz), 3.93-3.30 (m, 3H). ¹³CNMR (300 MHz, CDCl₃): δ = 169.75, 151.77, 150.15, 147.41, 144.09, 135.84, 130.61, 128.90, 128.87, 128.35, 126.79, 123.55, 119.13, 110.68, 108.10, 106.04, 103.45, 101.16, 101.02, 82.10, 78.20, 71.13^{*}, 71.11^{*}, 67.26, 61.13, 60.01, 57.91, 56.15, 55.76.

### Example 2: Preparation of Cleistanthin A acetate of Formula IV

Cleistanthin A acetate was prepared by the following reaction steps:

To a 50 mL round bottom flask, 9-(3',4'-Methylenedioxyphenyl)-4-hydroxy-6,7-dimethoxynaphtho[2,3-*c*]furan-1(3*H*)-one (formula II; 0.30 g, 0.788 mmole), 2-*O*-Acetyl-3,4-dimethoxy-a-D-bromoxylopyranose (formula III 0.446 g, 1.576 mmole) and tetrabutyl ammonium bromide (0.254 g, 0.788 mmole) were taken in dichloromethane (20 mL) with stirring. To this suspension was added 2M NaOH (3 mL) solution and stirring was continued for 2 h at room temperature. After the completion of reaction as judged by TLC (1:9, EtOAc:DCM), the reaction mixture was extracted with dichloromethane (4 x 20 mL). The combined organic layer washed with 10% NaOH solution (3 x 15 mL) followed by water (2 x 10 mL) and dried over anhydrous sodium sulfate. Inorganic salts were filtered off; filtrate was concentrated under reduced pressure and crude mass which was purified by column chromatography using EtOAc: dichloromethane (04:96) as eluent to Cleistanthin A acetate (formula IV) as white solid.

The yield and NMR details of compound of Cleistanthin A acetate were as follows: Yield: 0.240 g (52%)
¹HNMR (CDCl₃, 300 MHz): δ = 7.59 (s, 1H), 7.04 (s, 1H), 6.94 (d, 1H, *J* = 7.8 Hz), 6.81-6.76 (m, 2H), 6.06 (d, 1H, *J* = 13.8 Hz), 6.05 (d, 1H, *J* = 13.8 Hz), 5.47 (d, 1H, *J* = 16.2 Hz), 5.39 (d, 1H, *J* = 14.8 Hz), 5.33 (t, 1H, *J* = 7.2 Hz), 5.10 (d, 1H, *J* = 6.9 Hz), 4.15 (dd, 1H, *J* = 6.0, 13.2 Hz), 4.07 (s, 3H), 3.79 (s, 3H), 3.60 (s, 3H), 3.50 (s, 3H), 3.46-3.32 (m, 3H), 2.15 (s, 3H). ¹³CNMR (300 MHz, CDCl₃): δ = 169.68, 169.47, 151.83, 150.35, 147.51, 144.05, 135.57, 130.63, 128.37, 126.12, 125.94, 123.57, 119.25, 110.70, 108.20, 106.10, 101.21, 100.80, 100.58, 81.21^{**}, 81.16^{**}, 77.95, 71.44^{*}, 71.40^{*}, 66.94, 62.77, 59.90, 58.53, 56.22, 55.81, 21.14.
^{*} Signals of rotamers of same carbons due restricted rotation created by glycosidation.
^{**}Signals of rotamers of same carbons due restricted rotation created by glycosidation

### Example 3: Synthesis of 9-(3',4'-Methylenedioxyphenyl)-4-hydroxy-6,7-dimethoxynaphtho[2,3-c]-furan-1(3H)-one of Formula II

Compound of formula II was prepared by the following reaction steps:

### 1. Synthesis of 2-Bromo-4,5-dimethoxybenzaldehyde of formula VI

Three necked round bottom flask (500 mL) equipped with dropping funnel, magnetic stirrer, and stopper was charged with veratraldehyde or 4,5-dimethoxybenzaldehyde (formula V, 15 g, 0.090 mole) and acetic acid (210 mL). To this solution was added bromine (9.67 mL) in acetic acid (60 mL) dropwise with constant stirring over half an hour and stirring was further continued for 3 h at room temperature. During this time all the starting materials was consumed as confirmed by TLC (3: 7, EtOAc:Hexane). Water (250 mL) was added to the reaction mixture and cooled to 0°C. The precipitated solid was filtered off, washed with cold water and dried under vacuum to get a white solid 2-bromo-4,5-dimethoxybenzaldehyde (formula VI).

The yield and NMR details of compound of compound of formula VI were as follows: Yield: 19 g (85.85 %)
¹HNMR (CDCl₃, 300 MHz): δ = 10.19 (s, 1H), 7.43 (s, 1H), 7.07 (s, 1H), 3.97 (s, 3H), 3.93 (s, 3H).

### II. Synthesis of 2-(2-Bromo-4,5-dimethoxyphenyl)-1,3-dioxolane of formula VII

Three necked round bottom flask (250 mL) was equipped with Dean-Stark apparatus and reflux condenser, was charged with 2-bromo-4,5-dimethoxybenzaldehyde (formula VI, 19.0 g, 0.07 mole), toluene (200 mL), ethylene glycol (11.8 mL, 0.21 mole) and catalytic amount of *p*-toluene sulphonic acid (g, mmole). The reaction flask was immersed in oil bath and heated (90-95°C) under reflux for 9 h (till all the water removed). After completion of reaction as judged by TLC (2:8, EtOAc: Hexane), reaction mixture was allowed to cool to room temperature, neutralized by sodium bicarbonate solution and extracted with ethyl acetate (3 x 100 mL). All the organic layers were combined, dried over anhydrous sodium sulfate, filtered and concentrated under reduced pressure. The crude mass was purified by column chromatography over silica gel using ethyl acetate (5-10%) in hexane as eluent to afford 2-(2-bromo-4,5-dimethoxyphenyl)-1,3-dioxolane (formula VII) as a white solid.

The yield and NMR details of compound of compound of formula VII were as follows: Yield: 19.7 g (88 %)
¹HNMR (300 MHz, CDCl₃): δ = 7.11 (s, 1H), 7.01(s, 1H), 5.99(s, 1H), 4.18 (t, 2H, *J* = 6.9 Hz), 4.08 (t, 2H, *J*= 6.9 Hz), 3.89 (s, 3H), 3.88 (s, 3H).

### III. Synthesis of (2-(1,3-Dioxolan-2-yl)-4,5-dimelhoxyphenyl)(benzo[d][1,3]dioxol-5-yl)-methanol of formula VIII

To a flame dried three necked round bottom flask (100 mL) were added 2-(2-bromo-4,5-dimethoxyphenyl)-1,3-dioxolane (formula VII; 1.0 g, 0.0034 mole) and anhydrous THF (25 mL) under nitrogen atmosphere. The flask was cooling to -78°C in dry ice-acetone bath, *n*-BuLi (5.3 mL, 0.005 mole) was added dropwise with stirring at -78°C and stirred for 15 min. A separate flame dried flask was charged with piperonal (0.517 g, 0.0034 mole) and dry THF (6 mL). The piperonal solution was cannulated to the reaction mixture during 30 min and after the addition; reaction mixture was slowly warmed to room temperature and further stirred for 2.5 h. After the consumption of all bromo compound, as confirmed by TLC (50:50, EtOAc: Hexane), reaction mixture was quenched by the addition of saturated ammonium chloride solution and extracted with ethyl acetate (3 x 20 mL). All the organic layers were combined, dried over anhydrous sodium sulfate, filtered and concentrated. The crude product was purified by tituration with heptane and product (2-(1,3-Dioxolan-2-yl)-4,5-dimethoxyphenyl)(benzo[d][1,3]dioxol-5-yl)-methanol (formula VIII) (4) is sufficiently pure to proceed to next step.

The yield and NMR details of compound of compound of formula VIII were as follows: Yield: 1.00 g (83%)
¹HNMR (300 MHz, CDCl₃): δ = 7.14 (s, 1H), 6.90-6.78 (m, 4H), 6.11 (s, 1H), 5.96 (s, 2H), 5.90 (s, 1H), 4.19 (t, 2H, *J* = 6.6 Hz), 4.16 (t, 2H, *J* = 6.8 Hz), 4.02 (s, 3H), 3.81 (s, 3H), 3.17 (s, 1H). ¹³CNMR (300 MHz, CDCl₃): δ = 149.42, 148.11, 147.57, 146.58, 136.95, 135.43, 126.83, 121.04, 119.69, 111.48, 109.50, 107.92, 107.26, 101.65, 100.93, 71.34, 65.05, 55.94, 55.89.

### IV. Synthesis of Diethyl 1-(3',4'-methylenedioxyphenyl)-4-hydroxy-6,7-dimethoxy-naphthalene-2,3-dicarboxylate of formula IX

Sealed tube was charged with (2-(1,3-dioxolan-2-yl)-4,5-dimethoxyphenyl)(benzo[d][1,3]dioxol-5-yl)methanol (formula VIII, 0.30 g, 0.833 mmole), diethyl acetylinedicarboxylate (0.141 g, 0.833 mole), dichloromethane (0.4 mL) and glacial acetic acid (0.242 mL) and mixure was heated at 140 °C for 1 h. After completion of reaction as judged by TLC (50:50, EtOAc: Hexane), reaction mixture was cooled to room temperature, diluted with dichloromethane (10 mL),washed with 5 % sodium bicarbonate solution (3 x 10 mL), organic layer was dried over anhydrous sodium sulfate, filtered and concentrated. The crude reaction mass was purified by flash column chromatography over silica gel using EtOAc:hexane (15:85) to afford diethyl 1-(3',4'-methylenedioxyphenyl)-4-hydroxy-6,7-dimethoxynaphthalene-2,3-dicarboxylate (formula IX) as white solid.

The yield and NMR details of compound of compound of formula IX were as follows: Yield: 0.29 g (74 %)
¹HNMR (300 MHz, CDCl₃): δ = 7.73 (s, 1H), 6.89 (d, 1H, *J* = 7.8 Hz), 6.81-6.75 (m, 3H), 6.05 (d, 2H, *J*= 14.4 Hz), 4.44 (q, 2H, *J*= 7.2 Hz), 4.07 (q, 2H, *J*=6.9 Hz), 4.05 (s, 3H), 3.77 (s, 3 H), 1.38 (t, 3H, *J* = 7.2 Hz), 1.08 (t, 3H, *J =* 6.9 Hz). ¹³CNMR (300 MHz, CDCl₃): δ = 170.30, 168.74, 159.62, 152.37, 149.68, 147.22, 147.06, 132.21, 130.60, 128.99, 127.48, 124.37, 119.81, 111.42, 107.97, 105.73, 102.76, 101.09, 61.95, 60.81, 56.08, 55.79, 13.87, 13.82.

### V. Synthesis of 9-(3',4'-Methylenedioxyphenyl)-4-hydroxy-6,7-dimethoxynaphtho[2,3-c]furan-1(3H)-one of formula II

Two necked round bottom flask (25 mL) was charged with LAH (0.032 g, 0.852 mmol) and anhydrous THF (4 mL) and the mixture was cooled to 0°C with stirring. To this suspension, a solution of diethyl 1-(3',4'-methylenedioxyphenyl)-4-hydroxy-6,7-dimethoxynaphthalene-2,3-dicarboxylate (formula IX; 0.200 g, 0.426 mmol) in THF (4 mL) was added dropwise at 0°C and stirring was continued for 2 h at same temperature. After completion of reaction as judged by TLC (1:9, MeOH:DCM), reaction mixture was quenched with saturated sodium sulfate solution and extracted with *n*-butanol (4 x 20 mL). Organic layer was dried over anhydrous sodium sulfate, filtered and concentrated under reduced pressure. The crude residue was purified by flash column chromatography over silica gel to give yellow solid 9-(3',4'-Methylenedioxyphenyl)-4-hydroxy-6,7-dimethoxynaphtho[2,3-c]furan-1(3H)-one (formula II).

The yield and NMR details of compound of compound of formula II were as follows: Yield: 0.065 (80 %)
¹HNMR (300 MHz, DMSO-d₆) δ = 10.39 (s, 1H), 7.61 (s, 1H), 7.00 (d, 1H, *J* = 8.1 Hz), 6.94 (s, 1H), 6.85 (d, I H, *J* = 1.5 Hz), 6.75 (dd, 1H, *J* = 1.5, 8.4 Hz), 6.10 (s, 2H), 5.35 (s, 2H), 3.93 (s, 3H), 3.64 (s, 3H). ¹³CNMR (300 MHz, DMSO-d₆): δ = 169.81, 150.66, 149.89, 147.01, 146.76, 145.05, 129.71, 129.65, 128.95, 123.94, 123.45, 121.85, 118.86, 111.22, 108.02, 105.63, 101.19, 100.92, 66.71, 55.78, 55.29.

### Example 4: Synthesis of 2-O-Acetyl-3,4-dimethoxy-α-D-bromoxylopyranose of Formula III

Compound of formula III was prepared by the following reaction steps:

### I. Synthesis of Tetra-O- acetyl-D-xylopyranose of formula XI

To a three neck round bottam flask (500 mL), equipped with guard tube and stopper, were added D-xylose (formula X, 40.0 g, 0.266 mole), pyridine (200 mL) and cooled it at 0 °C. Acetic anhydride (200 mL) was added dropwise to the above mixture at 0 °C. The resulting reaction mixture was stirred at 0 °C for 5 h. After consumption of starting materials, as judged by TLC (5:5, EtOAc:Hexane), reaction mixture was poored into ice water (500 mL) and ether was added (500 L). Organic layer was separated and aqueous layer was extracted with ether (2 x 500 mL). Organic layers were combined and washed with saturated cupric salt solution till free from pyridine. The organic layer was dried over anhydrous sodium sulfate, filtered and concentrated to give sticky solid compound Tetra-O- acetyl-D-xylopyranose (formula XI).

The yield and NMR details of compound of compound of formula XI were as follows: Yield: 75 g (89 %)
¹HNMR (300 MHz, CDCl₃): δ = 6.27 (d, 1H, *J* = 3.6 Hz ), 5.70 (t, 1H, *J* = 9 Hz ), 5.06 (m, 2H), 3.97 (dd, 1H, *J* =6.0, 11.1 Hz), 3.72 (t, 1H, *J* = 11.0 Hz), 2.18 (s, 3H ), 2.07(s, 6H), 2.03 (s, 3H ).

### II. Synthesis of 2,3,4-Tri-O-acetyl-α-D-bromoxylopyranose of formula XII

1 L-round bottom flask with guard tube was charged tetra-O- acetyl-D-xylopyranose (formula XI; 25.0 g, 78.54 mole) and dichloromethane (500 mL) and mixture was cooled to 0 °C in ice bath. To the above cold solution was added hydrogen bromide (33 % in acetic acid; 56 mL) with constant stirring during 1 h and reaction mixture was further stirred at room temperature for 1 h. After completion of reaction as judged by TLC (4:6, EtOAc: Hexane), reaction mixture was washed with ice water (1 x 500 mL), I % NaHCO₃ solution (1 x 500 mL), 10 % NaHCO₃ solution (2 x 500 mL) and finally by brine solution (1 x 500 mL). Organic layer was dried over anhydrous sodium sulfate, filtered and concentrated under reduced pressure to obtained white solid 2,3,4-Tri-O-acetyl-α-D-bromoxylopyranose (formula XII), which was used directly in the next step.

The yield and NMR details of compound of compound of formula XII were as follows: Yield: 24.0 g (90 %)
¹HNMR (300 MHz, CDCl₃): δ = 6.59 (d, 1H, *J* = 3.9 Hz), 5.60 (t, 1H, *J* = 9.9 Hz), 5.05-5.03 (m, 1H), 4.77 (dd, 1H, *J* = 3.9, 9.6 Hz), 4.07 (dd, 1H, *J* = 6.3, 11.4 Hz), 3.88 (t, 1H, *J* = 11.1 Hz), 2.10 (s, 3H), 2.06 (s, 6H).

### III. Synthesis of 3,4-Di-O-acetyl-1,2-O-(1-ethoxyethylidene)-D-xylopyranose of formula XIII

Two necked round bottam flask were charged with 2,3,4-Tri-*O*-acetyl-α-D-bromoxylopyranose (formula XII ; 25.0 g, 73.71 mmole), 2,6-lutidine (11.07 mL , 95.82 mmol), tetrabutyl ammonium bromide (9.50 g, 29.48 mmole) and anhydrous dichloromethane (147 mL). To the above mixture was added absolute ethanol (4.7 mL, 81.08 mmole) and reaction mixture was stirred at room temperature under nitrogen atmosphere for overnight. After completion of reaction as judged by TLC (5:5, EtOAc: Hexane), the reaction mixture was concentrated under reduced pressure. The residue was purified by column chromatography over silica gel using EtOAc: Hexane as eluent to afford 3,4-Di-*O*-acetyl-1,2-*O*-(1-ethoxyethylidene)-D-xylopyranose (formula XIII) as a pale yellow colored liquid.

The yield and NMR details of compound of compound of formula XIII were as follows: Yield: 16.85 g (75 %)
¹HNMR (300 MHz, CDCl₃): δ = 5.57 (d, 1H, *J* =4.2 Hz), 5.24 (t, 1H, *J* = 3.6 Hz), 4.84-4.82 (m, 1H), 4.20 (t, 1H, *J* = 1.8 Hz), 3.89 (dd, 1H, *J =* 5.1, 12.3 Hz), 3.71 (dd, 1H, *J =* 6.9, 12.3 Hz), 3.59 (q, 2H, *J =* 6.9 Hz ), 2.10 (s, 3H), 2.08 (s, 3H), 1.19 (t, 3H, *J* = 6.9 Hz).

### IV. Synthesis of 1,2-O-(1-Ethoxyethylidene)-3,4-dimethoxy-D-xylopyranose of formula XIV

In a dried round bottam flask (250 mL) was charged with 3,4-Di-*O*-acetyl-1,2-*O*-(1-ethoxyethylidene)-D-xylopyranose (formula XIII; 10g, 32.86 mmole) and anhydrous methanol (157 mL) was added. To the above solution was added catalytic amount of sodium methoxide (300 mg) and stirred at room temperature for 1 h. After the completion of reaction as judged by TLC, reaction mixture was concentrated under reduced pressure and residue was dried under high vacuum. The resulting residue was dissolved in anhydrous DMF (100 mL) and cooled to 0°C in ice-bath. To the above cold solution, sodium hydride (3.94 g, 60% dispersion in oil, 164.3 mmole) was added and resulting suspension was with stirring for 1 h. Methyl iodide (12.4 mL, 197.6 mmole) was added dropwise at 0 °C, the reaction mixture was then slowly brought to room temperature during 1 h and further stirred at room temperature for 12 h. After completion of reaction, reaction was quenched by addition of methanol (10 mL), diluted with ethyl acetate (100 mL), washed with water (2 x 50 mL), brine solution (1 x 50 mL) and dried over anhydrous sodium sulfate. The inorganic salts were filtered off, filtrate was concentrated under reduced pressure and residue was purified by column chromatography using EtOAc:hexane (10:90) to afford 1,2-O-(1-Ethoxyethylidene)-3,4-dimethoxy-D-xylopyranose (formula XIV) as a light yellow colored liquid.

The yield and NMR details of compound of compound of formula XIV were as follows: Yield: 6.8g (83 %)
¹HNMR (300 MHz, CDCl₃): δ = 5.56 (d, 1H, *J* = 4.8 Hz), 4.29-4.26 (m, 1H), 3.89 (dd, 1H, *J* = 3.3, 12.1 Hz ), 3.82-3.69 (m, 5H ), 3.54 (s, 3H ), 3.44 (s, 3H), 3.26 (m, 1H), 1.19 (t, 3H, 6.9 Hz).

### V. Synthesis of 1,2-Di-O-acetyl-3,4-dimethoxy-D-xylopyranose of formula XV

1,2-*O*-(1-ethoxyethylidene)-3,4-dimethoxy-D-xylopyranose (formula XIV; 7.5 g, 30.20 mmole) was dissolved in acetic acid (55 mL) and resulting solution was stirred at 0 °C for 1 h. Reaction mixture was concentrated under reduced pressure and the residue was treated with acetic anhydride (26 mL) and pyridine (26 mL). The resulting solution was maintained at room temperature with stirring for overnight. After completion of reaction as judged by TLC (3:7, EtOAc:hexane ), reaction mixture was poored into cold water (100 mL) and extracted with ether (4 x 100 mL). The organic layers were combined, washed with saturated cupric sulfate solution till the pyridine was removed and then dried over anhydrous sodium sulfate. The inorganic solids were filtered off, filtrate was concentrated under reduced pressure and residue was purified by column chromatography over silica gel using EtOAc: hexane (20:80) as eluent to afford 1,2-Di-O-acetyl-3,4-dimethoxy-D-xylopyranose (formula XV) as a light yellow colored oil.

The yield and NMR details of compound of compound of formula XV were as follows: Yield: 5.0g (63 %)
¹HNMR (300 MHz, CDCl₃): δ = 5.62 (d, 1H, *J* = 7.2 Hz), 4.95 (t, 1H *J* = 7.8 Hz), 4.11 (m, 1H), 3.57 (s, 3H), 3.48(s, 3H), 3.39-3.31 (m, 3H), 2.10(s, 3H), 2.09(s, 3H).

### VI. Synthesis of 2-O-Acetyl-3,4-dimethoxy-α-D-bromoxylopyranose of formula III

In a clean and dry 50 mL-round bottam flask, 1,2-di-*O*-acetyl-3,4-dimethoxy-D-xylopyranose (formula XV; 1.0 g, 3.81 mmole) was dissolved in dichloromethane (25 mL) and cooled to 0oC in ice bath. To the above cooled solution was added hydrogen bromide in AcOH (33% solution; 2.5 mL) with constant stirring for 1 h and further stirred at room temperature for another 1 h. After completion of reaction as judged by TLC (3:7, EtOAc:Hexane), reaction mixture was diluted with dichloromethane (50 mL), washed with ice water (50 mL) followed by saturated NaHCO₃ solution (50 mL) and finally with brine solution (50 mL). Organic layer was dried over anhydrous sodium sulfate, filtered and concentrated to give yellow colored liquid 2-O-Acetyl-3,4-dimethoxy-α-D-bromoxylopyranose (formula III) as a product.

The yield and NMR details of compound of compound of formula III were as follows: Yield: 0.98g (90 %)
¹HNMR (300 MHz, CDCl₃): δ = 6.56 (d, 1H, *J* = 3.9 Hz), 4.56 (dd, 1H, *J* = 3.9, 9.6 Hz), 4.00 (dd, 1H, *J* = 6.3, 11.7. Hz), 3.72 (m, 1H), 3.56(s, 3H), 3.54 (s, 3H), 3.38 (m, 2H), 2.13 (s, 3H).

## Claims

1. A process for preparing compound of formula I, wherein the process comprises the steps of:
reacting compound of formula II with compound of formula III in the presence of a first solvent, quarternary ammonium salt and first alkali to form compound of formula IV;
treating compound of formula IV with a second alkali and a second solvent to form compound of formula I.

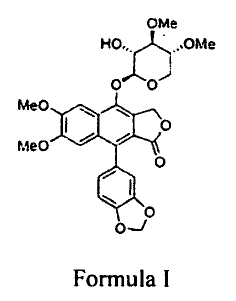

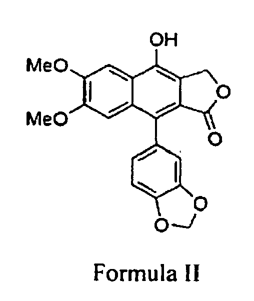

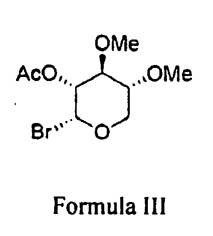

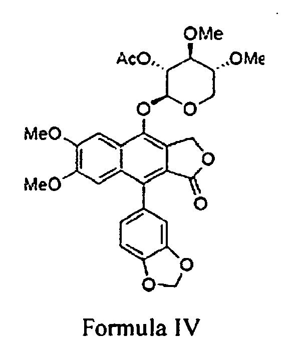

2. The process as claimed in claim 1 wherein the first solvent is dichloromethane.

3. The process as claimed in claim 1 wherein the first alkali is sodium hydroxide.

4. The process as claimed in claim 1 wherein the quarternary ammonium salt is tetrabutyl ammonium bromide.

5. The process as claimed in claim 1 wherein the second alkali is potassium carbonate.

6. The process as claimed in claim 1 wherein the second solvent is methanol.

7. A process for preparing compound of formula IV, wherein the process comprises the steps of:
reacting compound of formula II with compound of formula III in the presence of a solvent, quarternary ammonium salt and alkali to form compound of formula IV.

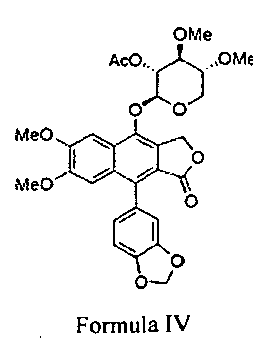

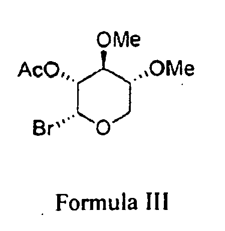

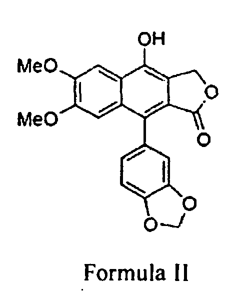

8. The process as claimed in claim 7 wherein the solvent is dichloromethane.

9. The process as claimed in claim 7 wherein the alkali is sodium hydroxide.

10. The process as claimed in claim 7 wherein the quarternary ammonium salt is tetrabutyl ammonium bromide.

11. The process as claimed in claim 1, wherein the compound of formula II is prepared by reducing compound of formula IX in the presence of lithium aluminium hydride and tetrahydrofuran.

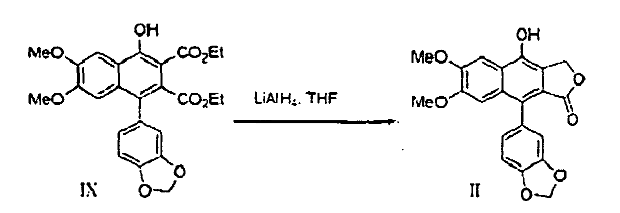

12. The process as claimed in claim 11 , wherein the compound of formula IX is prepared by reacting compound of formula VIII with diethyl acetylenedicarboxylate in the presence of acetic acid and methylene dichloride.

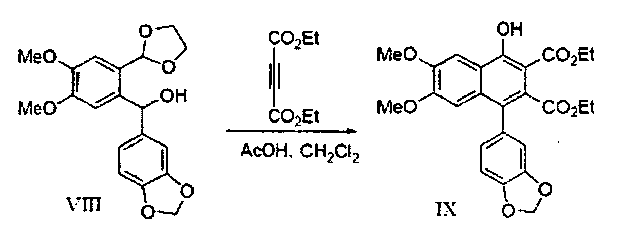

13. The process as claimed in claim 12, wherein the compound of formula VIII is prepared by treating compound of formula VII with n-butyl lithium in the presence of tetrahydrofuran and piperonal.

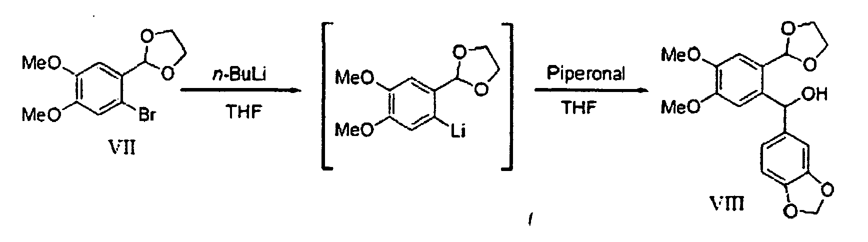

14. The process as claimed in claim 13, wherein the compound of formula VII is prepared by treating compound of formula VI with p-ethylene glycol in the presence of p-toluene sulphonic acid.

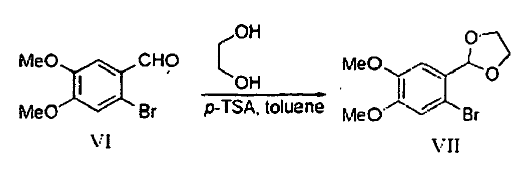

15. The process as claimed in claim 14, wherein the compound of formula VI is prepared by treating compound of formula V with bromine in the presence of acetic acid.

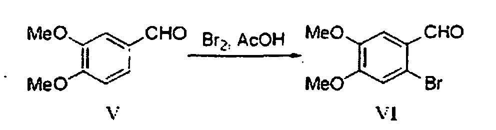

16. The process as claimed in claim 1, wherein the compound of formula III is prepared by reacting compound of formula XV with HBr in acetic acid in the presence of dichloromethane.

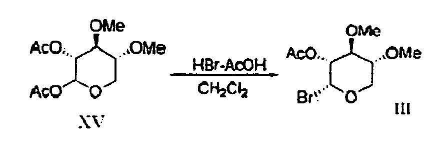

17. The process as claimed in claim 16 , wherein the compound of formula XV is prepared by treating compound of formula XIV with acetic acid in the presence of acetic anhydride and pyridine.

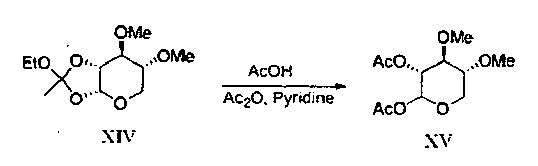

18. The process as claimed in claim 17, wherein the compound of formula XIV is prepared by treating compound of formula XIII with methanol and sodium methoxide to form a residue, which is further treated with dimethyl formamide in the presence of sodium hydride and methyl iodide.

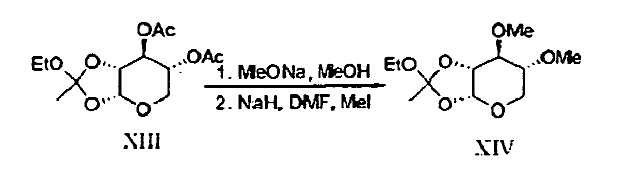

19. The process as claimed in claim 18, wherein the compound of formula XIII is prepared by treating compound of formula XII with 2,6 lutidine, tetrabutyl ammonium bromide, anhydrous dichloromethane and ethanol.

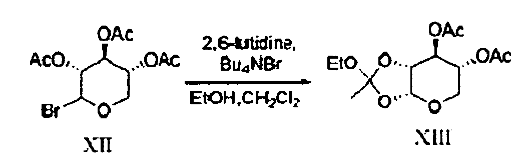

20. The process as claimed in claim 19, wherein the compound of formula XII is prepared by treating compound of formula XI with HBr in acetic acid in the presence of dichloromethane.

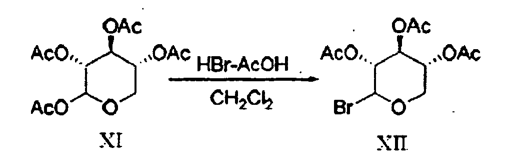

21. The process as claimed in claim 20, wherein the compound of formula XI is prepared by reacting compound of formula X with acetic anhydride in the presence of pyridine.

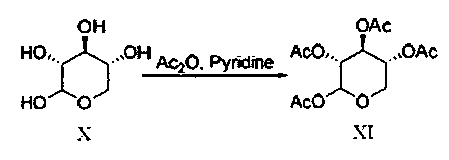

## Patentansprüche

1. Verfahren zur Herstellung einer Verbindung gemäß Formel 1, wobei das Verfahren die folgenden Schritte umfasst:
Umsetzen der Verbindung gemäß Formel II mit der Verbindung gemäß Formel III in Gegenwart von einem ersten Solvens, quartärem Ammoniumsalz und erstem Alkali zwecks Herstellung der Verbindung gemäß Formel IV;
Behandlung der Verbindung gemäß Formel IV mit einem zweiten Alkali und einem zweiten Solvens zur Herstellung gemäß Formel I.

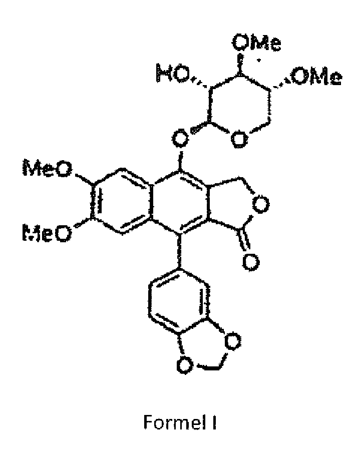

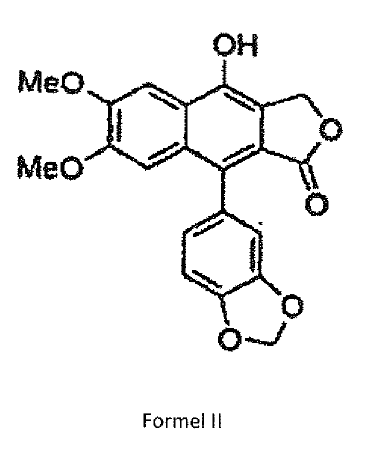

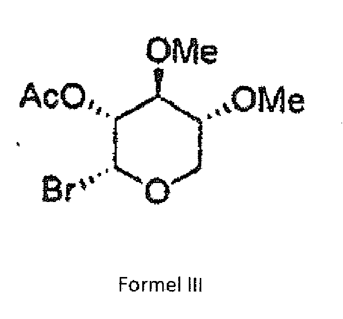

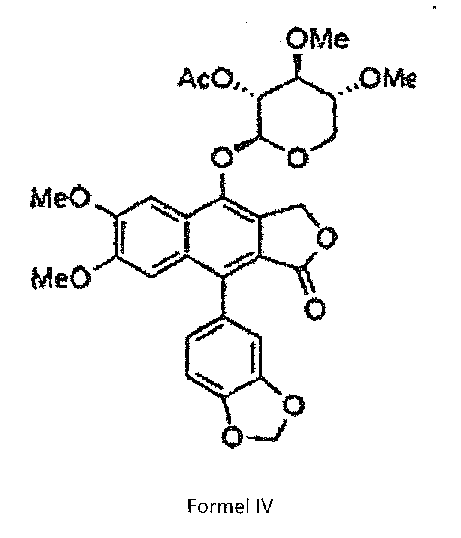

2. Verfahren gemäß Anspruch 1, wobei das erste Solvens Dichlormethan ist.

3. Verfahren gemäß Anspruch 1, wobei das erste Alkali Natriumhydroxid ist.

4. Verfahren gemäß Anspruch 1, wobei das quartäre Ammoniumsalz Tetrabutylammoniumbromid ist.

5. Verfahren gemäß Anspruch 1, wobei das zweite Alkali Kaliumkarbonat ist.

6. Verfahren gemäß Anspruch 1, wobei das zweite Solvens Methanol ist.

7. Verfahren zur Herstellung einer Verbindung gemäß Formel IV, wobei das Verfahren die folgenden Schritte umfasst:
Umsetzen der Verbindung gemäß Formel II mit der Verbindung gemäß Formel III in Gegenwart von einem Solvens, einem quartären Ammoniumsalz und einem Alkali zwecks Herstellung der Verbindung gemäß Formel IV.

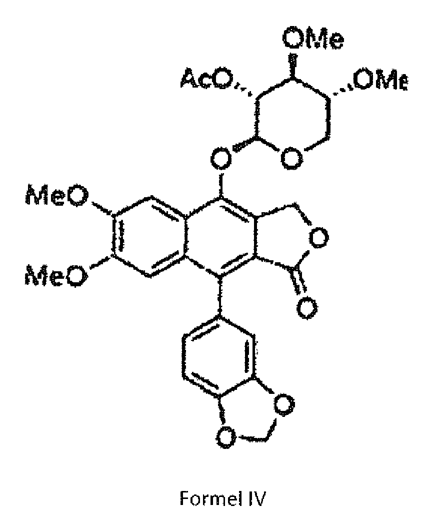

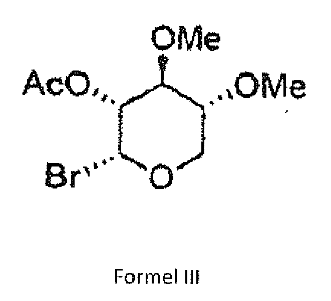

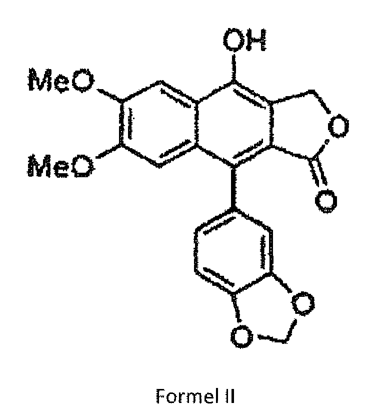

8. Verfahren gemäß Anspruch 7, wobei das Solvens Dichlormethan ist.

9. Verfahren gemäß Anspruch 7, wobei das Alkali Natriumhydroxid ist.

10. Verfahren gemäß Anspruch 7, wobei das quartäre Ammoniumsalz Tetrabutylammoniumbromid ist.

11. Verfahren gemäß Anspruch 1, wobei die Verbindung gemäß Formel II durch Reduktion der Verbindung gemäß Formel IX in Gegenwart von Lithiumaluminiumhydrid und Tetrahydrofuran hergestellt wird.

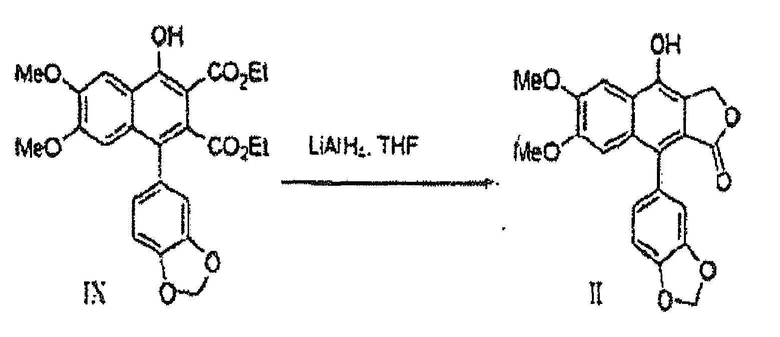

12. Verfahren gemäß Anspruch 11, wobei die Verbindung gemäß Formel IX durch Umsetzen der Verbindung gemäß Formel VIII mit Acethylen-dicarbonsäurediethylester in Gegenwart von Essigsäure und Methylendichlorid hergestellt wird.

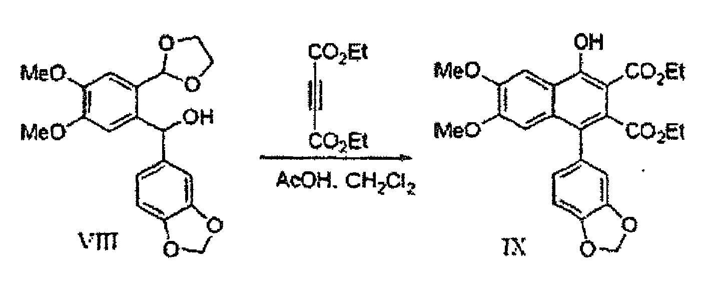

13. Verfahren gemäß Anspruch 12, wobei die Verbindung gemäß Formel VIII durch Behandlung der Verbindung gemäß Formel VII mit n-Butyllithium in Gegenwart von Tetrahydrofuran und Piperonal hergestellt wird.

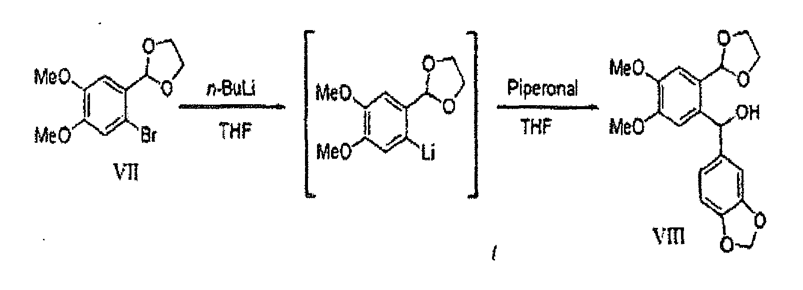

14. Verfahren gemäß Anspruch 13, wobei die Verbindung gemäß Formel VII durch Behandlung der Verbindung gemäß Formel VI mit p-Ethylenglykol in Gegenwart von p-Toluolsulfonsäure hergestellt wird.

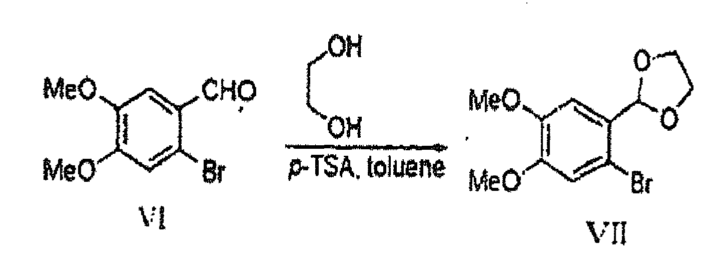

15. Verfahren gemäß Anspruch 14, wobei die Verbindung gemäß Formel VI durch Behandlung der Verbindung gemäß Formel V mit Brom in Gegenwart von Essigsäure hergestellt wird.

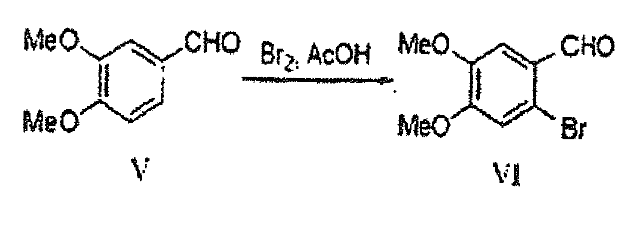

16. Verfahren gemäß Anspruch 1, wobei die Verbindung gemäß Formel III durch Umsetzen der Verbindung gemäß Formel XV mit HBr in Essigsäure in Gegenwart von Dichlormethan hergestellt wird.

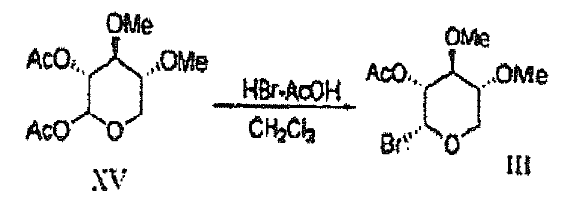

17. Verfahren gemäß Anspruch 16, wobei die Verbindung gemäß Formel XV durch Behandlung der Verbindung gemäß Formel XIV mit Essigsäure in Gegenwart von Essigsäureanhydrid und Pyridin hergestellt wird.

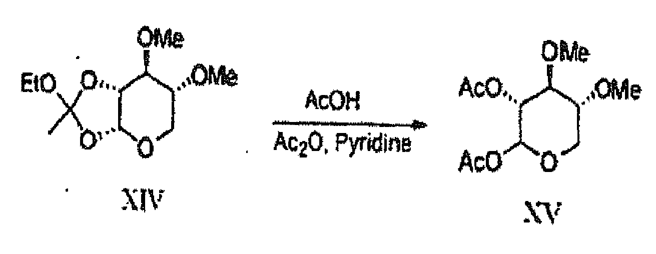

18. Verfahren gemäß Anspruch 17, wobei die Verbindung gemäß Formel XIV durch Behandlung der Verbindung gemäß Formel XIII mit Methanol und Natriummethanolat hergestellt wird und einen Rückstand bildet, welcher mit Dimethylformamid in Gegenwart von Natriumhydrid und lodmethan weiter behandelt wird.

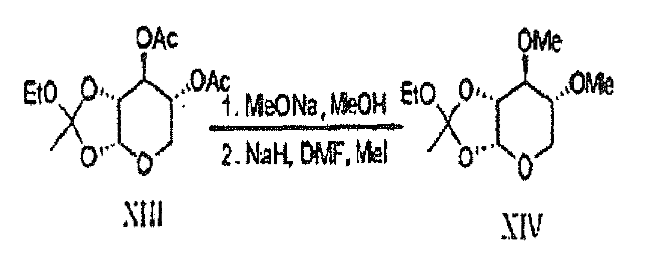

19. Verfahren gemäß Anspruch 18, wobei die Verbindung gemäß Formel XIII durch Behandlung der Verbindung gemäß Formel XII mit 2,6-Lutidin, Tetrabutylammoniumbromid, wasserfreiem Dichlormethan und Ethanol hergestellt wird.

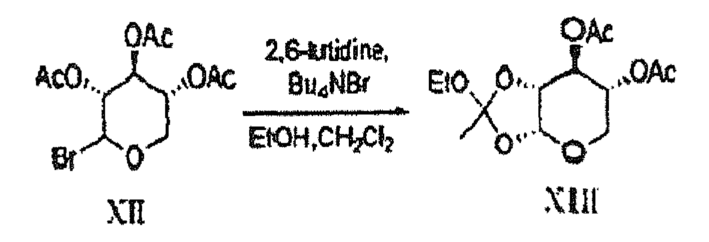

20. Verfahren gemäß Anspruch 19, wobei die Verbindung gemäß Formel XII durch Behandlung der Verbindung gemäß Formel XI mit HBr in Essigsäure in Gegenwart von Dichlormethan hergestellt wird.

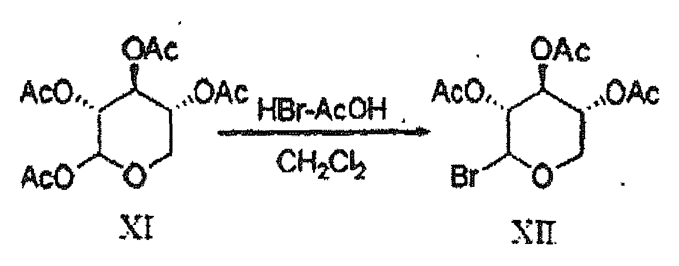

21. Verfahren gemäß Anspruch 20, wobei die Verbindung gemäß Formel XI durch Umsetzen der Verbindung gemäß Formel X mit Essigsäureanhydrid in Gegenwart von Pyridin hergestellt wird.

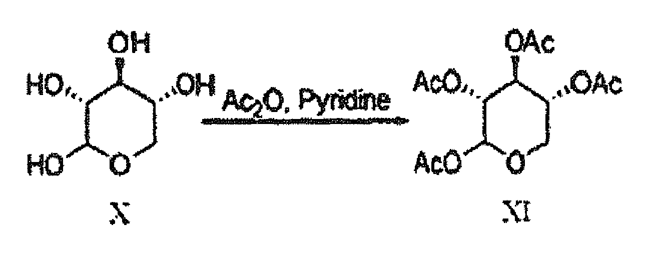

## Revendications

1. Procédé de préparation d'un composé selon la formule 1, le procédé comprenant les étapes suivantes:
Faire réagir le composé selon la formule 1 avec le composé selon la formule III en présence d'un premier solvant, de sel d'ammonium quaternaire et d'un premier alcali pour produire un composé selon la formule IV;
Traiter le composé selon la formule IV avec un deuxième alcali et un deuxième solvant pour produire un composé selon la formule I.

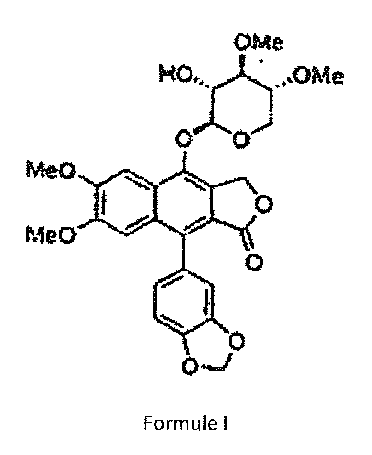

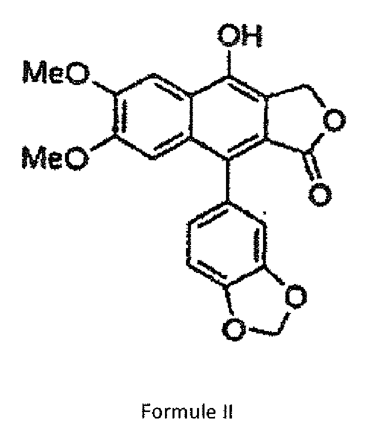

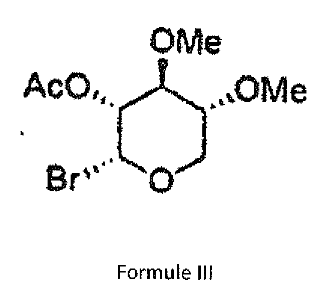

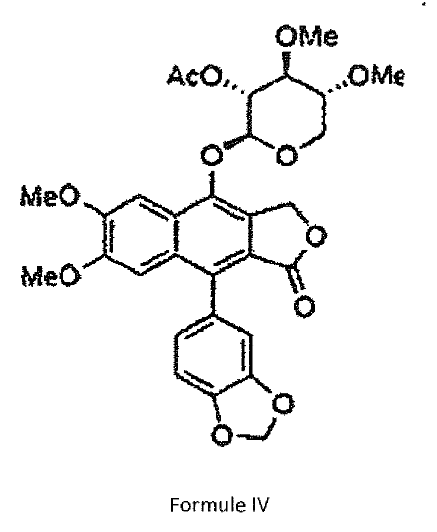

2. Le procédé selon la revendication 1, **caractérisé en ce que** le premier solvant est du dichlorométhane.

3. Le procédé selon la revendication 1, **caractérisé en ce que** le premier alcali est de l'hydroxyde de sodium.

4. Le procédé selon la revendication 1, **caractérisé en ce que** le sel d'ammonium quaternaire est du bromure de tétrabutylammonium.

5. Le procédé selon la revendication 1, **caractérisé en ce que** le deuxième alcali est du carbonate de potassium.

6. Le procédé selon la revendication 1, **caractérisé en ce que** le deuxième solvant est du méthanol.

7. Le procédé de préparation d'un composé selon la formule IV, le procédé comprenant les étapes suivantes:
Faire réagir le composé selon la formule II avec le composé selon la formule III en présence d'un solvant, de sel d'ammonium quaternaire et d'un alcali pour produire un composé selon la formule IV.

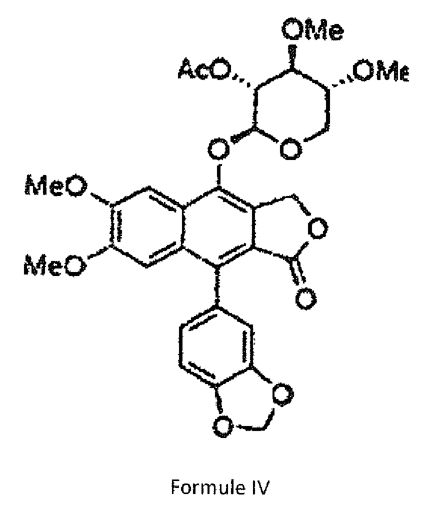

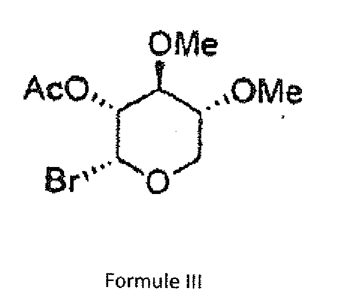

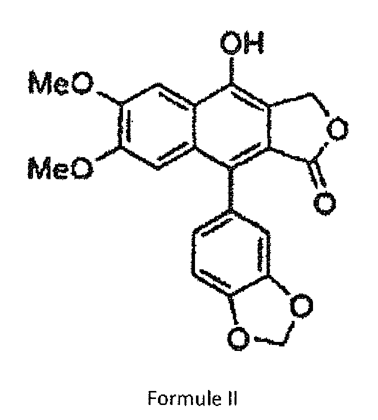

8. Le procédé selon la revendication 7, **caractérisé en ce que** le solvant consiste en du dichlorométhane.

9. Le procédé selon la revendication 7, **caractérisé en ce que** l'alcali est de l'hydroxyde de sodium.

10. Le procédé selon la revendication 7, **caractérisé en ce que** le sel d'ammonium quaternaire est du bromure de tétrabutylammonium.

11. Le procédé selon la revendication 1, **caractérisé en ce que** le composé selon la formule II est préparé en réduisant le composé selon la formule IX en présence d'hydruroaluminate de lithium et de tétrahydrofurane.

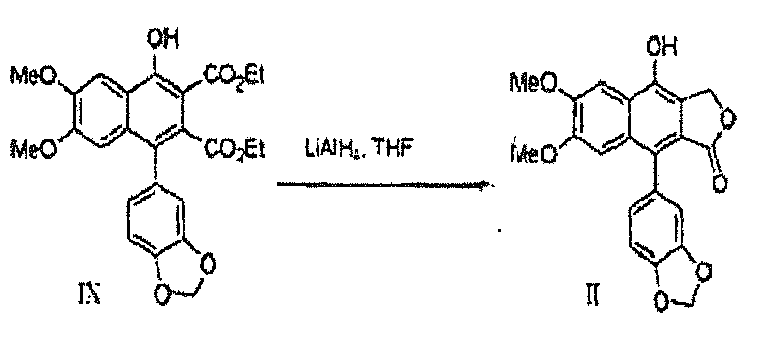

12. Le procédé selon la revendication 11, **caractérisé en ce que** le composé selon la formule IX est préparé en faisant réagir le composé selon la formule VIII avec de l'éthynedicarboxylate de diéthyle en présence d'acide acétique et de dichlorométhane.

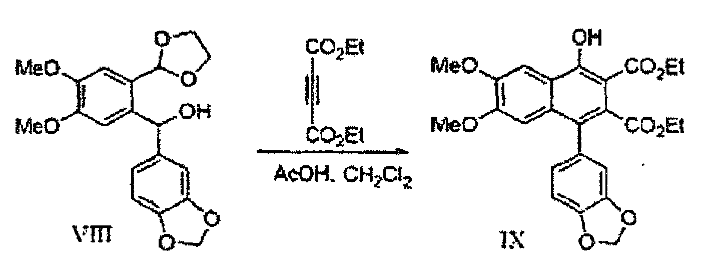

13. Le procédé selon la revendication 12, **caractérisé en ce que** le composé selon la formule VIII est préparé en traitant le composé selon la formule VII avec du n-butyillithium en présence de tétrahydrofurane et de pipéronal.

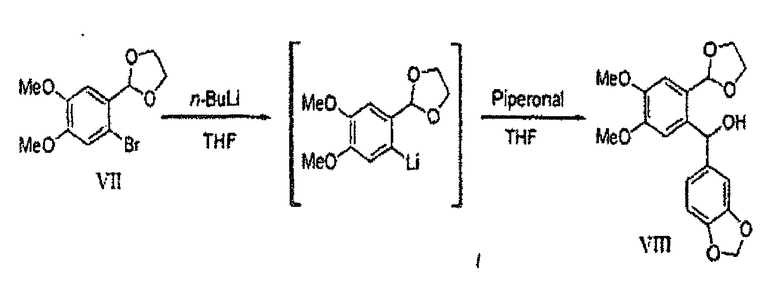

14. Le procédé selon la revendication 13, **caractérisé en ce que** le composé selon la formule VII est préparé en traitant le composé selon la formule VI avec du p-éthylène glycol en présence d'acide p-toluène sulfonique.

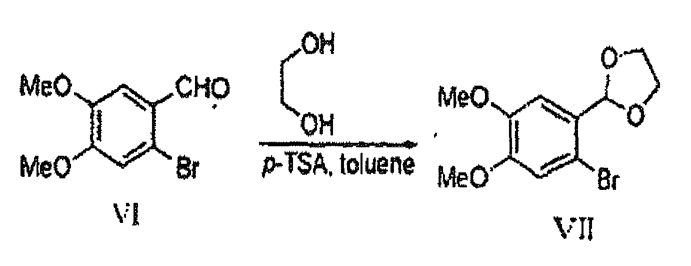

15. Le procédé selon la revendication 14, **caractérisé en ce que** le composé selon la formule VI est préparé en traitant le composé selon la formule V avec du brome en présence d'acide acétique.

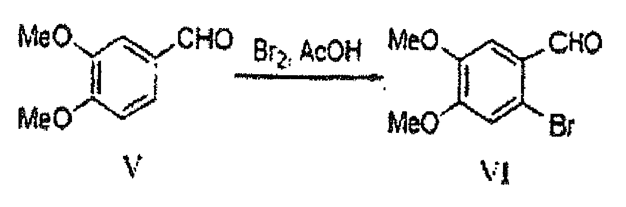

16. Le procédé selon la revendication 1, **caractérisé en ce que** le composé selon la formule III est préparé en faisant réagir le composé selon la formule XV avec du HBr dans l'acide acétique en présence de dichlorométhane.

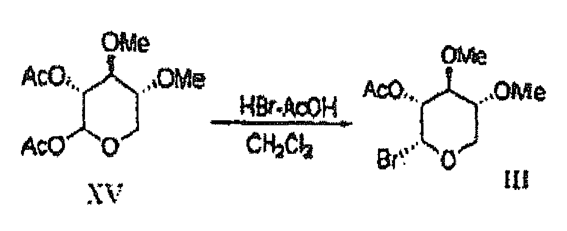

17. Le procédé selon la revendication 16, **caractérisé en ce que** le composé selon la formule XV est préparé en traitant le composé selon la formule XIV avec de l'acide acétique en présence d'anhydride acétique et de pyridine.

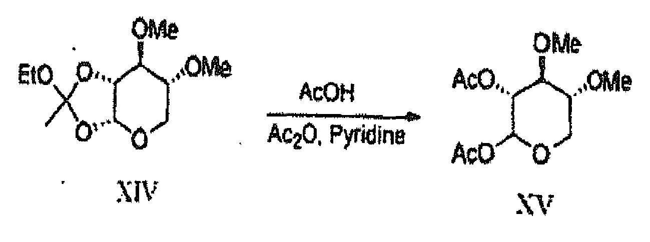

18. Le procédé selon la revendication 17, **caractérisé en ce que** le composé selon la formule XIV est préparé en traitant le composé selon la formule XIII avec du méthanol et du méthylate de sodium pour produire un résidu, lequel est ensuite traité avec du diméthylformamide en présence d'hydrure de sodium et d'iodure de méthyle.

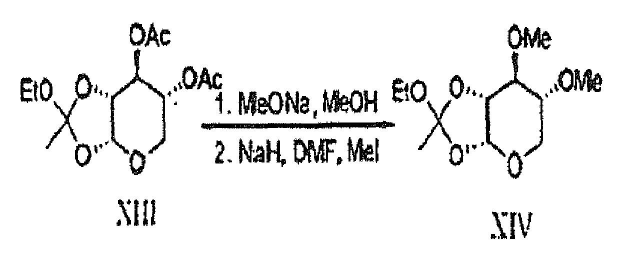

19. Le procédé selon la revendication 18, **caractérisé en ce que** le composé selon la formule XIII est préparé en traitant le composé selon la formule XII avec de la 2,6-lutidine, du bromure de tétrabutylammonium, du dichlorométhane anhydre et de l'éthanol.

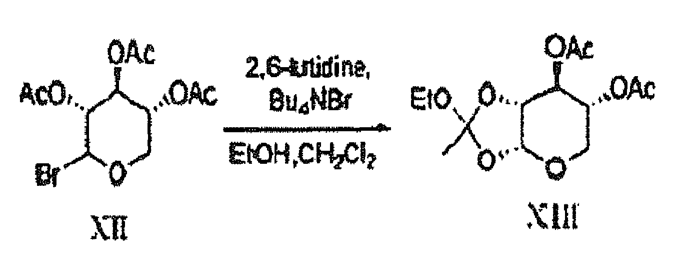

20. Le procédé selon la revendication 19, **caractérisé en ce que** le composé selon la formule XII est préparé en traitant le composé selon la formule XI avec du HBr dans l'acide acétique en présence de dichlorométhane.

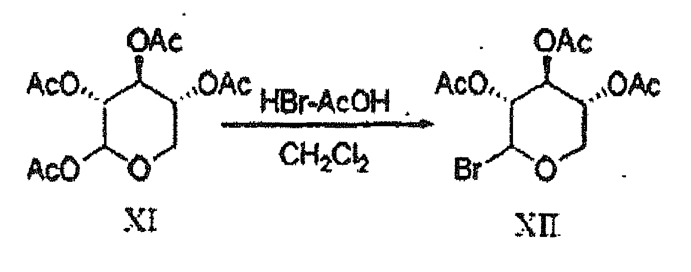

21. Le procédé selon la revendication 20, **caractérisé en ce que** le composé selon la formule XI est préparé en faisant réagir le composé selon la formule X avec de l'anhydride acétique en présence de pyridine.
